# DEMANDE DE BREVET EUROPEEN

(11) **EP 1 258 265 A1**
(43) Date de publication de la demande: **20.11.2002**
(21) Numéro de dépôt: 02291197.8
(22) Date de dépôt: 14.05.2002
(51) Int. Cl.: A61N 1/372

(54) **Procédé de gestion de données médicales d'un dispositif implantable actif tel que stimulateur cardiaque, défibrillateur, cardioverteur et/ou dispositif multisite, à l'usage d'un cardiologue**

(30) Priorité: 15.05.2001 FR 0106374
(71) Demandeur: ELA MEDICAL, F-92541 Montrouge (FR)
(72) Inventeur: Graindorge, Laurence, 92290 Chatenay Malabry (FR); Limousin, Marcel, 75014 Paris (FR)
(74) Mandataire: Dupuis-Latour, Dominique

(57) **Abrégé**

Ce procédé comprend les étapes consistant à : lire par télémétrie la mémoire de données (12) du dispositif (10) d'un patient au moyen d'un programmateur (14); mémoriser dans une base de données du programmateur les données médicales brutes ainsi lues, dans un champ mémoire (24) contenant un identifiant du patient (26), une adresse de cardiologue ou médecin traitant (28) assurant le suivi médical du patient, ainsi que lesdites données médicales brutes (30) ; transmettre par voie télématique (36) audit cardiologue ou médecin traitant, à l'adresse qui lui correspond, les données médicales relatives au patient identifié ; recueillir, traiter et présenter au cardiologue ou médecin traitant les données médicales ainsi transmises au moyen d'un logiciel dédié (44) implémenté sur un micro-ordinateur (40) à disposition de ce cardiologue ou médecin traitant.

## Description

L'invention concerne les "dispositifs médicaux implantables actifs" tels que définis par la Directive 90/385/CE du 20 juin 1990 du Conseil des communautés européennes. Cette définition inclut les stimulateurs cardiaques, défibrillateurs, cardioverteurs et/ou dispositifs multisite, mais aussi les appareils neurologiques, les pompes de diffusion de substances médicales, les implants cochléaires, les capteurs biologiques implantés, etc., ainsi que les dispositifs de mesure de pH ou encore de mesure d'impédance intracorporelle.

Ces dispositifs, par la suite dénommés simplement "implants", comportent une mémoire de données qui peut être lue au moyen d'un programmateur externe par des techniques de télémétrie en elles-mêmes bien connues. Le programmateur est associé à un micro-ordinateur à la disposition du praticien et comportant un écran d'affichage, un clavier pour l'entrée de commandes et la saisie de données, ainsi que divers moyens de mémorisation et de traitement de données.

Le praticien utilisateur du programmateur est un médecin spécialisé, qui est généralement celui qui a implanté le dispositif ou bien qui travaille en étroite coopération avec ce dernier, dans le même centre hospitalier. Il maîtrise parfaitement le fonctionnement du programmateur et a un accès direct aux données stockées dans la mémoire de l'implant, ainsi qu'aux diverses commandes de "programmation", c'est-à-dire les commandes susceptibles de modifier le paramétrage et le fonctionnement du stimulateur.

Le point de départ de l'invention est le constat que la mémoire de l'implant contient des données susceptibles d'intéresser un autre praticien, qui est un autre spécialiste ou bien le médecin traitant qui assure le suivi courant du patient.

Si l'on prend l'exemple d'un implant cardiaque, le premier médecin spécialisé est le spécialiste dénommé "électrophysiologiste" ou "rythmologue", et le second médecin est le cardiologue ou le médecin traitant qui assure le suivi courant du patient.

Le cardiologue dispose certes du rapport de l'électrophysiologiste, qui inclut des interprétations liées au fonctionnement de l'implant et éventuellement au rythme cardiaque ; mais l'état clinique du patient implique de mettre l'accent sur d'autres paramètres, notamment pour soigner les pathologies, prescrire une médication, etc., tâches propres au cardiologue et qui ne sont pas du ressort de l'électrophysiologiste et pour lesquelles le cardiologue dispose du rapport de l'électrophysiologiste et des résultats des examens cliniques qu'il effectue lui-même ou fait effectuer.

Toutefois, il existe certaines données dont la connaissance pourrait être intéressante pour l'aider à poser son diagnostic, données préexistantes, stockées dans la mémoire de l'implant.

Généralement, le cardiologue ne dispose cependant pas d'un programmateur qui lui permettrait de lire la mémoire de l'implant, ce qui supposerait d'ailleurs qu'il soit formé à l'utilisation d'un tel programmateur, appareil qui est susceptible par ailleurs de modifier la programmation du stimulateur. De plus, la lecture de l'ensemble des données brutes de la mémoire implique un travail de sélection et d'analyse qui rend en pratique difficile l'utilisation courante d'un programmateur par un cardiologue.

Le but de l'invention est de remédier à cette difficulté, en proposant un procédé de gestion de données médicales issues de la mémoire de l'implant, propre à fournir au cardiologue des informations lui permettant d'aider à poser son diagnostic et à établir une prescription, sans pour autant nécessiter l'acquisition d'un matériel supplémentaire.

On notera que l'invention n'est pas limitée à l'exemple préférentiel que l'on expose ici, et qui a trait au domaine de la cardiologie (avec la dualité électrophysiologiste/cardiologue ou électrophysiologiste/médecin traitant), mais s'applique *mutatis mutandis* à d'autres domaines médicaux impliquant des intervenants avec des compétences distinctes ayant besoin chacun en ce qui les concerne, en des lieux et à des moments différents, d'informations particulières contenues dans l'implant.

Le procédé de l'invention est d'un type connu, par exemple d'après le EP-A-0 761 255, comprenant les étapes consistant à : (a) lire par télémétrie la mémoire de données du dispositif d'un patient au moyen d'un programmateur ; (b) mémoriser dans une base de données du programmateur les données médicales brutes ainsi lues, dans un champ mémoire contenant un identifiant du patient ainsi que lesdites données médicales brutes ; (c) transmettre par voie télématique les données médicales relatives au patient identifié ; et (d) recueillir, traiter et présenter les données médicales ainsi transmises.

De façon caractéristique de l'invention : (1°) l'étape b comprend également la mémorisation dans ledit champ mémoire d'une adresse d'un cardiologue ou d'un médecin traitant assurant le suivi médical du patient ; (2°) l'étape c est une transmission audit cardiologue ou médecin traitant, à l'adresse qui lui correspond, des données médicales relatives au patient identifié ; et (3°) l'étape d comprend le recueil, le traitement et la présentation audit cardiologue ou médecin traitant des données médicales ainsi transmises, au moyen d'un logiciel dédié implémenté sur un micro-ordinateur à disposition de ce cardiologue ou médecin traitant.

Dans un mode de mise en oeuvre particulier, l'étape c de transmission est une étape de transmission en temps différé comprenant les sous-étapes de : (c1) transmission, à un serveur tiers, des données médicales relatives au patient identifié avec leur adresse de cardiologue ou médecin traitant associée ; (c2) mémorisation, dans une base de données du serveur tiers, desdites données médicales avec leur adresse associée ; et (c3) téléchargement ultérieur de ces données médicales par ledit cardiologue ou médecin traitant identifié par ladite adresse.

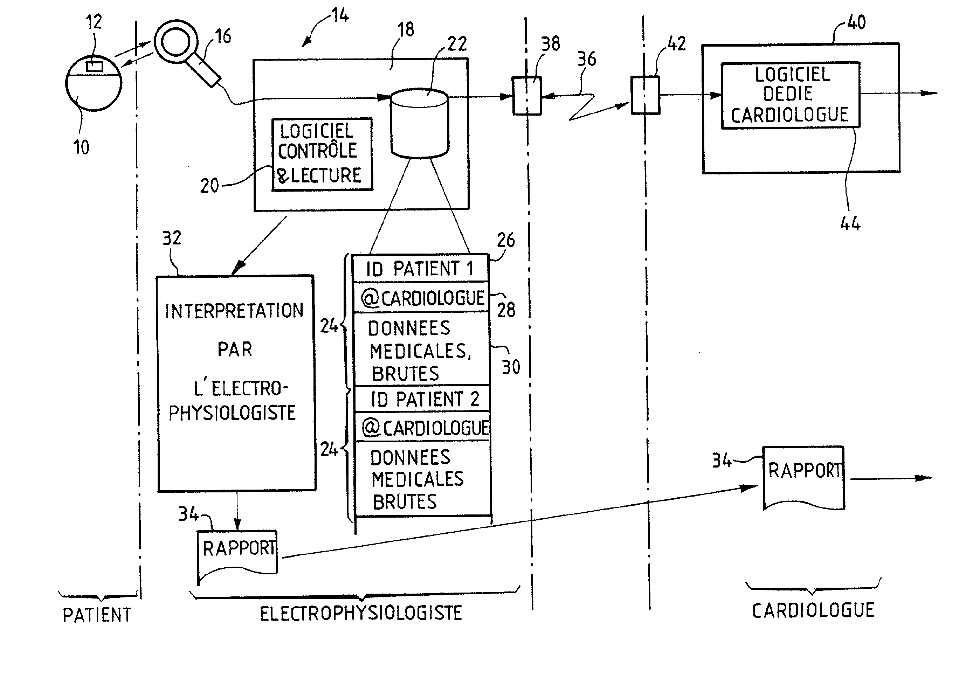

On va maintenant décrire un exemple de mise en oeuvre du procédé de l'invention, en référence à la figure unique annexée qui représente schématiquement l'ensemble des éléments mis en oeuvre dans le cadre de ce procédé.

Comme on l'a indiqué plus haut, notamment dans le domaine de la cardiologie, le besoin existe d'offrir aux médecins cardiologues, qui soignent les pathologies du patient (médication, insuffisance cardiaque) mais qui ne contrôlent pas le stimulateur et n'ont donc pas accès aux données stockées par celui-ci, un certain nombre d'informations leur permettant de les aider dans leur diagnostic.

On notera que l'invention ne concerne pas la manière dont sont élaborées ces données, qui préexistent dans la mémoire de l'implant, ni le diagnostic proprement dit, qui est posé par le praticien à l'aide des données qui lui sont fournies ainsi que d'autres informations dont il dispose sur le patient et sa pathologie.

Dans l'exemple illustré, l'implant 10 est un stimulateur cardiaque ou un défibrillateur capable de stocker dans sa mémoire interne 12 des données médicales tels que des électrogrammes sur les arythmies auriculaires ou ventriculaires, l'évolution de la ventilation du patient, son activité, sa fréquence ventriculaire, les apnées du sommeil, le niveau du seuil de capture, des valeurs d'impédance intracardiaque ou autre, etc. Ces informations brutes sont disponibles dans la mémoire, mais jusqu'à présent réservées à l'électrophysiologiste car lui seul dispose d'un matériel permettant de les lire, et ne seront donc pas accessibles au cardiologue ou médecin traitant assurant le suivi courant du patient.

Pour lire les données stockées dans la mémoire 12 de l'implant 10, l'électrophysiologiste dispose d'un programmateur 14 avec une tête de télémétrie 16 susceptible d'établir une liaison de communication avec l'implant et un micro-ordinateur 18 assurant les diverses fonctions de traitement et de mémorisation des données recueillies par la tête de télémétrie.

Le micro-ordinateur 18 inclut notamment un logiciel 20 de lecture des données et de contrôle (programmation) de l'implant, et des moyens de mémorisation 22.

De façon caractéristique de l'invention, lorsque l'électrophysiologiste interroge la mémoire 12 de l'implant lors d'un contrôle, le logiciel 20 va, en sus des fonctions habituelles, créer un fichier informatique spécifique comportant une pluralité de zones 24 (une pour chaque patient interrogé) avec pour chacun un champ 26 d'identification du patient, un champ 28 d'adresse électronique du cardiologue ou médecin traitant assurant le suivi de ce patient, et un champ 30 stockant une sélection de données médicales brutes qui pourront être utiles dans le cadre d'un examen clinique du patient.

Ces données sont conservées dans la base de données du programmateur pour utilisation ultérieure.

Par ailleurs, de manière en elle-même classique, l'électrophysiologiste effectue son travail d'interprétation sur les troubles du rythme, le synchronisme, etc. (travail schématisé par le bloc 32), interprétation concrétisée par l'établissement d'un rapport 34, papier ou électronique, qui sera adressé au médecin traitant ou cardiologue.

Lors de la prochaine consultation de son patient, le médecin traitant ou le cardiologue va interroger la base de données 22 via une liaison télématique 36, par exemple une liaison Internet, l'ordinateur 18 de l'électrophysiologiste étant raccordé à cette liaison par un modem correspondant 38. Le cardiologue peut ainsi télécharger des données dans son ordinateur 40, qui est un matériel de type courant, non dédié, simplement doté d'un modem 42.

On notera que la transmission de données de l'ordinateur 18 de l'électro-physiologiste à l'ordinateur 40 du cardiologue peut se faire aussi bien à l'initiative de l'électrophysiologiste qu'à celle du cardiologue.

Par exemple, après acquisition des données brutes l'électrophysiologiste peut envoyer, à chaque cardiologue identifié de chaque patient, un message électronique comprenant (en fichier joint ou incorporée au message) une copie de ces données brutes mémorisées dans la base de données 22.

Dans ce dernier cas, la transmission peut être opérée systématiquement lors de la consultation chez l'électrophysiologiste, tandis que dans l'autre cas l'ordinateur de l'électrophysiologiste est interrogé par le cardiologue à l'occasion d'une consultation chez ce dernier.

L'existence dans le fichier 24 du champ 28 contenant l'adresse du cardiologue permet d'identifier ce dernier et de réserver à lui seul la transmission des données médicales 30 qui lui sont destinées.

Les données brutes ainsi téléchargées pourront être consultées par l'intermédiaire d'un logiciel dédié 44 chargé dans l'ordinateur 40, pour permettre par exemple un affichage des données médicales sous forme de tableau ou de pages écran. Le logiciel 44 est un logiciel dédié aux cardiologues, très simple dans la mesure où il ne contient que des commandes de téléchargement des données et de présentation de celles-ci sur l'écran de l'ordinateur.

A partir de ces données ainsi que du rapport 34 de l'électrophysiologiste, le cardiologue pourra bénéficier de toutes les informations disponibles sur son patient afin de poser au mieux un diagnostic et d'établir une prescription appropriée.

Dans un mode de mise en oeuvre particulier, un service tiers tel qu'un serveur d'hébergement de site internet peut faire fonction d'intermédiaire de liaison entre les ordinateurs de l'électrophysiologiste et du cardiologue. Le tiers fournisseur de service peut ainsi conserver une base de données reproduisant le contenu de la base de données 22 du programmateur. Dans cette mise en oeuvre, les données brutes d'un patient donné sont envoyées par l'électrophysiologiste à la base de données du tiers, où elles sont stockées et toujours accessibles au cardiologue. Le cardiologue peut alors se connecter au serveur tiers et récupérer par téléchargement les données spécifiques du patient concerné. De cette manière le cardiologue est indépendant de l'ordinateur de l'électrophysiologiste 18, qui n'a pas besoin d'être actif et accessible à distance pour permettre le téléchargement.

## Revendications

1. Un procédé de gestion de données médicales issues de la mémoire d'un dispositif médical implantable actif tel que stimulateur cardiaque, défibrillateur, cardioverteur et/ou dispositif multisite,
ce procédé comprenant les étapes consistant à :
a) lire par télémétrie la mémoire de données (12) du dispositif (10) d'un patient au moyen d'un programmateur (14),
b) mémoriser dans une base de données du programmateur les données médicales brutes ainsi lues, dans un champ mémoire (24) contenant un identifiant du patient (26) ainsi que lesdites données médicales brutes (30),
c) transmettre par voie télématique (36) les données médicales relatives au patient identifié, et
d) recueillir, traiter et présenter les données médicales ainsi transmises,
procédé **caractérisé en ce que** :
- l'étape b) comprend également la mémorisation dans ledit champ mémoire (24) d'une adresse d'un cardiologue ou d'un médecin traitant (28) assurant le suivi médical du patient,
- l'étape c) est une transmission audit cardiologue ou médecin traitant, à l'adresse qui lui correspond, des données médicales relatives au patient identifié, et
- l'étape d) comprend le recueil, le traitement et la présentation audit cardiologue ou médecin traitant des données médicales ainsi transmises, au moyen d'un logiciel dédié (44) implémenté sur un micro-ordinateur (40) à disposition de ce cardiologue ou médecin traitant.

2. Le procédé de la revendication 1, dans lequel l'étape c) de transmission est une étape de transmission en temps différé comprenant les sous-étapes de :
c1) transmission, à un serveur tiers, des données médicales relatives au patient identifié avec leur adresse de cardiologue ou médecin traitant associée,
c2) mémorisation, dans une base de données du serveur tiers, desdites données médicales avec leur adresse associée, et
c3) téléchargement ultérieur de ces données médicales par ledit cardiologue ou médecin traitant identifié par ladite adresse.
